(19) Europäisches Patentamt
European Patent Office
Office européen des brevets

(11) Veröffentlichungsnummer: **0 415 212 A1**

(12) **EUROPÄISCHE PATENTANMELDUNG**

(21) Anmeldenummer: **90115874.1**

(22) Anmeldetag: **18.08.90**

(51) Int. Cl.[5]: **C07C 69/712**, C07C 323/50, A01N 39/00

(30) Priorität: **01.09.89 DE 3928988**

(43) Veröffentlichungstag der Anmeldung: **06.03.91 Patentblatt 91/10**

(84) Benannte Vertragsstaaten: **BE CH DE DK FR GB IT LI NL**

(71) Anmelder: **BAYER AG**
**W-5090 Leverkusen 1 Bayerwerk(DE)**

(72) Erfinder: **Andree, Roland, Dr.**
**Schillerstrasse 17**
**W-4018 Langenfeld(DE)**
Erfinder: **Haug, Michael, Dr.**
**Fahner Weg 5**
**W-5060 Bergisch Gladbach 2(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Aryloxynaphthalinderivate.**

(57) Neue Aryloxynaphthalinderivate der allgemeinen Formel (I)

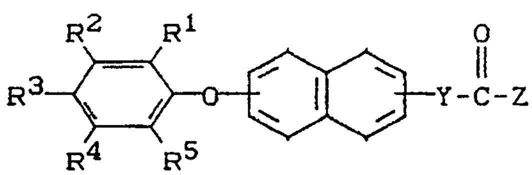

(I)

in welcher
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht,
$R^5$ für Alkoxy oder Alkylthio steht,
Y für eine der Gruppierungen -A-, -O-A- oder -S-A-
steht, wobei
A für gegebenenfalls durch Halogen substituiertes Alkandiyl steht, und

EP 0 415 212 A1

Z für Hydroxy, Alkoxy, Alkenyloxy, Alkinyloxy, Halogenalkoxy, Alkoxyalkoxy, Aralkoxyalkoxy, Alkoxycarbonylalkoxy, Arylalkoxy, Amino, Alkylamino, Alkenylamino, Alkinylamino, Dialkylamino, Dialkenylamino, Dialkinylamino, Alkoxyalkylamino oder Alkoxycarbonylalkylamino steht,
Verfahren zu ihrer Herstellung und ihre Verwendung als Herbizide.

## ARYLOXYNAPHTHALINDERIVATE

Die Erfindung betrifft neue Aryloxynaphthalinderivate, mehrere Verfahren zu ihrer Herstellung und ihre Verwendung als Herbizide.

Es ist bekannt, daß bestimmte Aryloxynaphthalinderivate, wie z.B. α-(7-(2-Chlor-4-trifluormethyl-phenoxy)-naphthalin-2-yl-oxy)-propionsäure-ethylester, herbizide Eigenschaften aufweisen (vgl. EP-A 179015). Die Wirkung dieser bekannten Verbindungen gegen Unkräuter ist jedoch nicht in allen Belangen zufriedenstellende.

Es wurden nun neue Aryloxynaphthalinderivate der allgemeinen Formel (I)

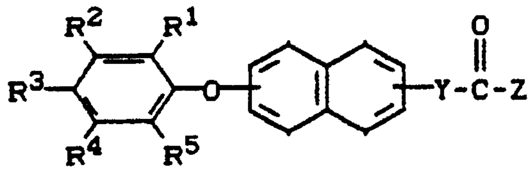

(I)

in welcher
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht,
$R^5$ für Alkoxy oder Alkylthio steht,
Y für eine der Gruppierungen -A-, -O-A- oder -S-A-
steht, wobei
A für gegebenenfalls durch Halogen substituiertes Alkandiyl steht, und
Z für Hydroxy, Alkoxy, Alkenyloxy, Alkinyloxy, Halogenalkoxy, Alkoxyalkoxy, Aralkoxyalkoxy, Alkoxycarbonylalkoxy, Arylalkoxy, Amino, Alkylamino, Alkenylamino, Alkinylamino, Dialkylamino, Dialkenylamino, Dialkinylamino, Alkoxyalkylamino oder Alkoxycarbonylalkylamino steht,
gefunden

Im Falle eines asymmetrischen Kohlenstoffatoms im Rest A betrifft die Erfindung sowohl die Isomerengemische als auch die reinen R- und S-Isomere bevorzugt die racemischen Gemische und die R-Isomeren.

Weiter wurde gefunden, daß man die neuen Aryloxynaphthalinderivate der allgemeinen Formel (I) ernalt, wenn man

(a) Halogenaryloxynaphthalinderivate der allgemeinen Formel (II)

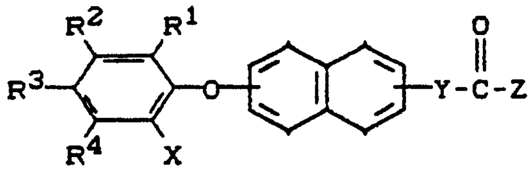

(II)

in welcher
$R^1$, $R^2$, $R^3$, $R^4$, Y und Z die oben angegebenen Bedeutungen haben und
X für Halogen steht,
mit Alkanolen oder Alkanthiolen und/oder mit deren Alkalimetallsalzen gegebenenfalls in Gegenwart eines Säureakzeptors und gegebenenfalls in Gegenwart eines Verdünnungsmittels umsetzt, oder wenn man
(b) für den Fall daß in der Formel (I) Y für eine der Gruppierungen -O-A- oder -S-A- steht und $R^1$, $R^2$, $R^3$, $R^4$, $R^5$ und Z die oben angegebenen Bedeutungen haben,
Aryloxynaphthaline der allgemeinen Formel (III)

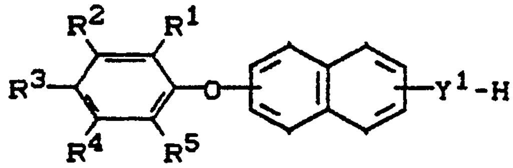

(III)

in welcher
$R^1$, $R^2$, $R^3$, $R^4$ und $R^5$ die oben angegebenen Bedeutungen haben und
$Y^1$ für Sauerstoff oder Schwefel steht,
mit Carbonsäurederivaten der allgemeinen Formel (IV)

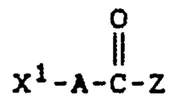

(IV)

in welcher
A und Z die oben angegebenen Bedeutungen haben und
$X^1$ für eine nucleophile Abgangsgruppe steht,
gegebenenfalls in Gegenwart eines Säureakzeptors und gegebenenfalls in Gegenwart eines Verdünnungsmittels umsetzt.

Schließlich wurde gefunden, daß die neuen Aryloxynaphthalinderivate der allgemeinen Formel (I) interessante herbizide Eigenschaften aufweisen.

Überraschenderweise zeigen die erfindungsgemäßen Aryloxynaphthalinderivate der Formel (I) erheblich starkere Herbizidwirkung als die bekannte Verbindung α-(7-(2-Chlor-4-trifluormethyl-phenoxy)naphthalin-2-yl-oxy) propionsäure-ethylester, welches ein strukturell ähnlicher vorbekannter Wirkstoff gleicher Wirkungsrichtung ist.

Die Kohlenstoffketten in den Kohlenwasserstoffgruppierungen, wie beispielsweise Alkyl, Alkenyl, Alkinyl, Alkoxy, Alkenyloxy, Alkinyloxy, Alkylamino Alkenylamino, Alkinylamino oder Alkandiyl, sind jeweils geradkettig oder verzweigt.

Die Erfindung betrifft vorzugsweise Verbindungen der Formel (I), in welcher
$R^1$ für Wasserstoff, Fluor, Chlor, Brom, Cyano oder Trifluormethyl steht,
$R^2$ für Wasserstoff, Fluor oder Chlor steht,
$R^3$ für Fluor, Chlor, Brom, Trifluormethyl, Trifluormethoxy, Trifluormethylthio oder Trifluormethylsulfonyl steht,
$R^4$ für Wasserstoff Fluor oder Chlor steht,
$R^5$ für $C_1$-$C_4$-Alkoxy oder $C_1$-$C_4$-Alkylthio steht,
Y für eine der Gruppierungen -O-A- oder -S-A-
steht, wobei
A für gegebenenfalls durch Fluor, Chlor und/oder Brom substituiertes $C_1$-$C_4$-Alkandiyl steht, und
Z für Hydroxy, $C_1$-$C_6$-Alkoxy, $C_3$-$C_6$-Alkenyloxy, $C_3$-$C_6$-Alkinyloxy, $C_1$-$C_6$-Halogenalkoxy, $C_1$-$C_4$-Alkoxy-$C_1$-$C_4$-alkoxy, Benzyloxy-$C_2$-$C_3$-alkoxy, $C_1$-$C_4$-Alkoxycarbonyl-$C_1$-$C_3$-alkoxy, Benzyloxy, Amino, $C_1$-$C_6$-Alkylamino, $C_3$-$C_6$-Alkenylamino, $C_3$-$C_6$-Alkinylamino, Di-($C_1$-$C_4$-alkyl)-amino, Di-($C_3$-$C_4$-alkenyl)-amino, Di-($C_3$-$C_4$-alkinyl)-amino, $C_1$-$C_4$-Alkoxy-$C_1$-$C_4$-alkylamino oder $C_1$-$C_4$-Alkoxy-carbonyl-$C_1$-$C_3$-alkylamino steht.

Die Erfindung betrifft insbesondere Verbindungen der Formel (I), in welcher
$R^1$ für Chlor steht,
$R^2$ für Wasserstoff steht,
$R^3$ für Trifluormethyl steht,
$R^4$ für Wasserstoff steht,
$R^5$ für Methoxy, Ethoxy, Propoxy, Methylthio oder Ethylthio steht,
Y für eine der Gruppierungen -$A^1$-, -O-$A^2$- oder -S-$A^2$-
steht, wobei
$A^1$ für gegebenenfalls durch Chlor substituiertes Ethan-1,2-diyl steht und
$A^2$ für Methylen oder Ethan-1,1-diyl (Ethyliden) steht, und
Z für Hydroxy, Methoxy, Ethoxy, Propoxy, Isopropoxy, Butoxy, sec-Butoxy, Isobutoxy, tert-Butoxy, Methoxyethoxy, Ethoxyethoxy, Methoxycarbonylmethoxy, Ethoxycarbonylmethoxy, Methoxycarbonylethoxy, Ethoxycarbonylethoxy, Benzyloxy, Benzyloxyethoxy, Amino, Methylamino, Ethylamino, Propylamino, Isopropylamino, Butylamino, Isobutylamino, sec-Butylami no, tert-Butylamino Methoxyethylamino, Ethoxyethylamino, Methoxycarbonylmethylamino, Ethoxycarbonylmethylamino, Methoxycarbonylethylamino oder Ethoxycarbonylethylamino steht.

Ganz besonders bevorzugte Gruppen von Verbindungen der Formel (I) ind diejenigen der nachstehenden Formeln (IA) bis (IF), in welchen jeweils $R^1$, $R^2$, $R^3$, $R^4$, $R^5$, Y und Z die oben als insbesondere bevorzugt angegebenen Bedeutungen haben Insbesondere sei die Gruppe (IB) hervorgehoben.

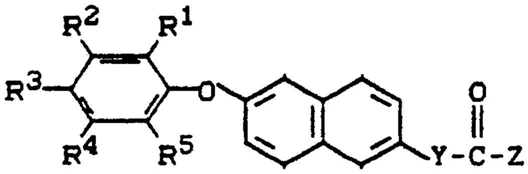

(IA)

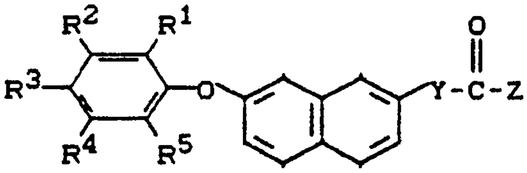

(IB)

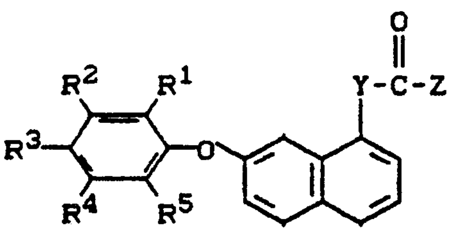

(IC)

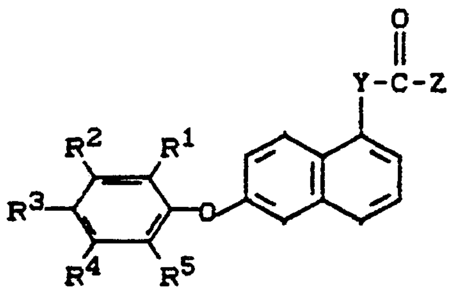

(ID)

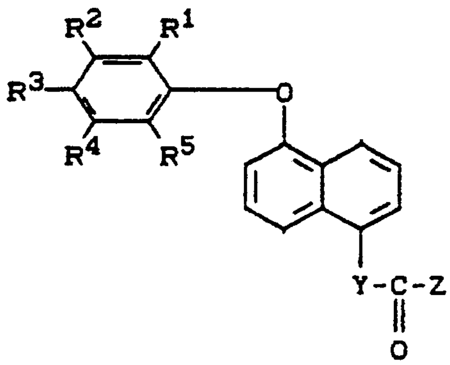

(IE)

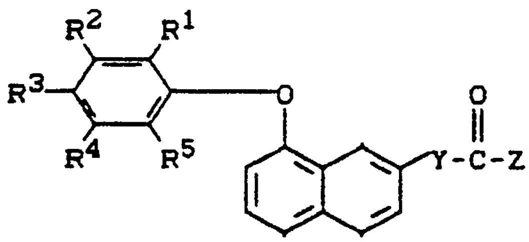

(IF)

Beispielhaft sind die in der nachstehenden Tabelle 1 aufgeführten Verbindungen der Formel (I) genannt:

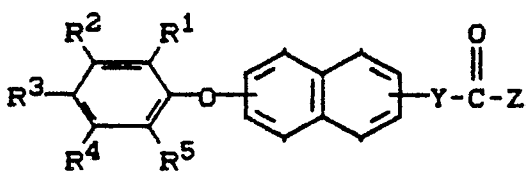

(I)

**Tabelle 1**

| $R^1$ | $R^2$ | $R^3$ | $R^4$ | $R^5$ | Y | Z |
|---|---|---|---|---|---|---|
| Cl | H | $CF_3$ | H | $OCH_3$ | $-O-CH_2-$ | OH |
| Cl | H | $CF_3$ | H | $OCH_3$ | $-O-CH_2-$ | $OCH_3$ |
| Cl | H | $CF_3$ | H | $OCH_3$ | $-O-CH_2-$ | $OC_2H_5$ |
| Cl | H | $CF_3$ | H | $OC_2H_5$ | $-O-CH_2-$ | OH |
| Cl | H | $CF_3$ | H | $OC_2H_5$ | $-O-CH_2-$ | $OCH_3$ |
| Cl | H | $CF_3$ | H | $OC_2H_5$ | $-O-CH_2-$ | $OC_2H_5$ |
| Cl | H | $CF_3$ | H | $OCH_3$ | $-O-CH(CH_3)-$ | OH |
| Cl | H | $CF_3$ | H | $OCH_3$ | $-O-CH(CH_3)-$ | $OCH_3$ |
| Cl | H | $CF_3$ | H | $OCH_3$ | $-O-CH_2-$ | $OC_4H_9$ |
| Cl | H | $CF_3$ | H | $OCH_3$ | $-S-CH_2-$ | OH |
| Cl | H | $CF_3$ | H | $OCH_3$ | $-S-CH_2-$ | $OCH_3$ |
| Cl | H | $CF_3$ | H | $OCH_3$ | $-S-CH(CH_3)-$ | $OCH_3$ |
| Cl | H | $CF_3$ | H | $OC_2H_5$ | $-S-CH_2-$ | $OC_2H_5$ |
| Cl | H | $CF_3$ | H | $OCH_3$ | $-S-CH(CH_3)-$ | OH |
| Cl | H | $CF_3$ | H | $SCH_3$ | $-O-CH_2-$ | OH |
| Cl | H | $CF_3$ | H | $SCH_3$ | $-O-CH_2-$ | $OCH_3$ |
| Cl | H | $CF_3$ | H | $SCH_3$ | $-O-CH_2-$ | $OCH(CH_3)_2$ |

**Tabelle 1 - Fortsetzung**

| $R^1$ | $R^2$ | $R^3$ | $R^4$ | $R^5$ | Y | Z |
|---|---|---|---|---|---|---|
| Cl | H | $CF_3$ | H | $SCH_3$ | $-S-CH_2-$ | OH |
| Cl | H | $CF_3$ | H | $SCH_3$ | $-S-CH-$<br>$CH_3$ | $OCH_3$ |
| Cl | H | $CF_3$ | H | $OCH_3$ | $-CH_2CH_2-$ | OH |
| Cl | H | $CF_3$ | H | $OCH_3$ | $-CH_2CH_2-$ | $OCH_3$ |
| Cl | H | $CF_3$ | H | $OCH_3$ | $-CH_2-CH-$<br>Cl | $OCH_3$ |

Die in Tabelle 1 angegebenen Beispiele gelten im einzelnen für die Gruppen von Verbindungen der Formel (I), welche durch die Formeln (IA) bis (SF) skizziert sind.

Verwendet man für das erfindungsgemäße Verfahren (a) beispielsweise α-(7-(2,6-Dichlor-4-trifluormethyl-phenoxy}-naphthalin-2-yl-oxy)-propionsäureethylester und Natriummethylat sie Ausgangsstoffe so kann der Reaktionsablauf durch das folgende Formelschema wiedergegeben werden:

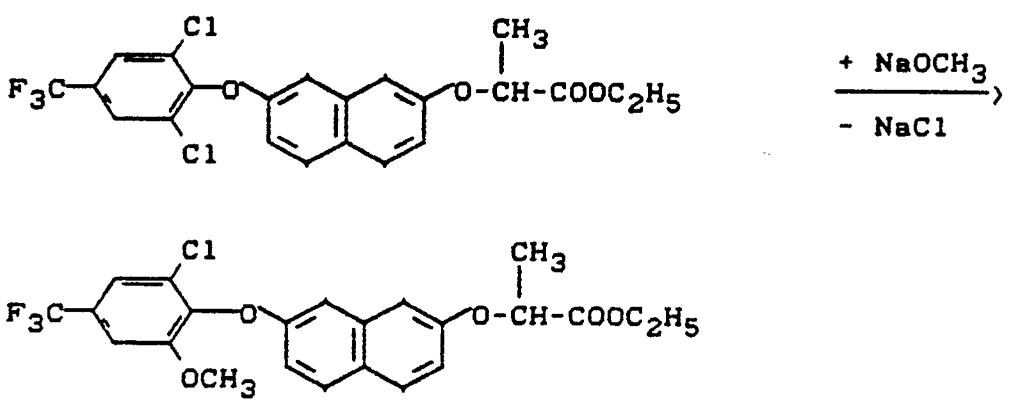


Verwendet man für das erfindungsgemäße Verfahren (b) beispielsweise 6-(2-Chlor-6-methylthio-4-trifluormethylphenoxy)-2-naphthol und Bromessigäure-methylester als Ausgangsstoffe, so kann der Reaktionsablauf durch das folgende Formelschema wiedergegeben werden:

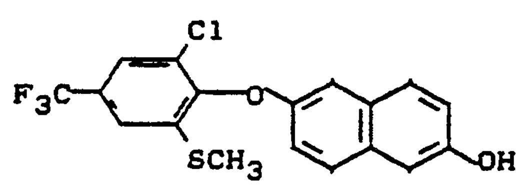

+ $Br-CH_2-COOCH_3$

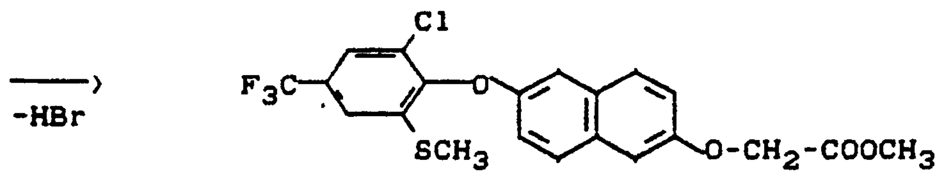


Die beim erfindungsgemaßen Verfahren (a) zur Herstellung von Verbindungen der Formel (I) als Ausgangsstoffe zu verwendenden Halogenaryloxynaphthalinderivate sind durch die Formel (II) allgemein definiert

In Formel (II) haben $R^1$, $R^2$, $R^3$, $R^4$, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, Y und Z angegeben wurden und

X steht vorzugsweise für Fluor, Chlor oder Brom, insbesondere für Chlor oder Fluor.

Die Ausgangsstoffe der Formel (II) sind bekannt und/oder Gegenstand von vorgängigen Patentanmeldungen (vgl. EP-A 179 015, EP-A 308 755, EP-A 309 864, EP-A 315 008, DE-P 3 823 318 vom 09.07.1988, DE-P 3 821 389 vom 24.06.1988 oder DE-P 3 837 464 vom 04.11.1988).

Die noch nicht vorveröffentlichten Ausgangsstoffe der Formel (II), in welcher Y für die Gruppierung -S-A-steht und $R^1$, $R^2$, $R^3$, $R^4$, A, X und 2 die oben angege benen Bedeutungen haben (vgl. auch DE-P 3 823 318vom 09.07.1988) erhält man beispielsweise, wenn man

in einer ersten Stufe Halogenbenzol-Derivate der allgemeinen Formel (V)

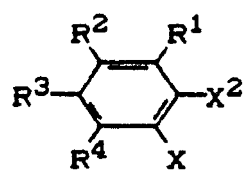
(V)

in welcher

$R^1$, $R^2$, $R^3$, $R^4$ und X die oben angegebenen Bedeutungen haben und

$X^2$ für Halogen, vorzugsweise Fluor oder Chlor, steht, mit Hydroxynaphthalinsulfonsäuren der allgemeinen Formel (VI)

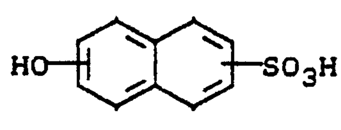
(VI)

oder mit deren Metallsalzen gegebenenfalls in Gegenwart von Säureakzeptoren, wie z.B. Natrium- oder Kaliumhydroxid, und gegebenenfalls in Gegenwart von Verdünnungsmitteln, wie z.B. Dimethylformamid oder Dimethylsulfoxid, bei Temperaturen zwischen 20 °C und 120 °C umsetzt, die so erhaltenen Aryloxynaphthalinsulfonsäure-Derivate der allgemeinen Formel (VII)

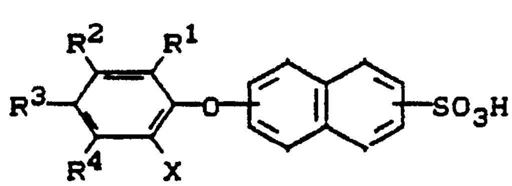
(VII)

in welcher

$R^1$, $R^2$, $R^3$, $R^4$ und X die oben angegebenen Bedeutungen haben,

oder deren Metallsalze

in einer zweiten Stufe mit Halogenierungsmitteln, wie z.B. Phosphor(V)-chlorid und/oder Phosphorylchlorid (Phosphoroxychlorid), bei Temperaturen zwischen 0 °C und 100 °C umsetzt, die so erhaltenen Aryloxynaphthalinsulfonsäurechloride der allgemeinen Formel (VIII)

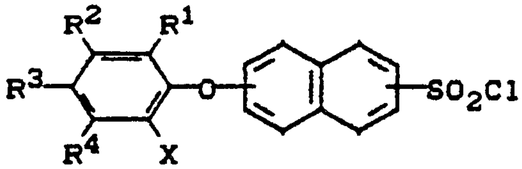

(VIII)

in welcher
$R^1$, $R^2$, $R^3$, $R^4$ und X die oben angegebenen Bedeutungen haben,
in einer dritten Stufe mit Reduktionsmitteln, wie z.B. Zinkstaub, gegebenenfalls in Gegenwart von Reduktionshilfsmitteln, wie z.B. Salzsäure oder Essigsäure, und gegebenenfalls in Gegenwart eines Verdünnungsmittels, wie z.B. Dioxan, bei Temperaturen zwischen 10 °C und 120 °C umsetzt, und die so erhaltenen Aryloxynaphthalinthiole der allgemeinen Formel (IXa)

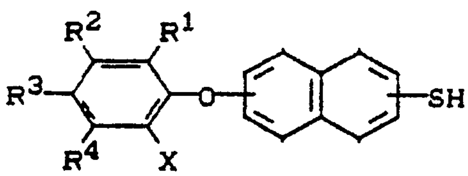

(IXa)

in welcher
$R^1$, $R^2$, $R^3$, $R^4$ und X die oben angegebenen Bedeutungen haben,
in einer vierten Stufe mit Carbonsäurederivaten der allgemeinen Formel (IV)

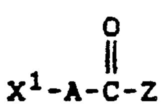

(IV)

in welcher
A, $X^1$ und Z die oben angegebenen Bedeutungen haben
gegebenenfalls in Gegenwart eines Säureakzeptors, wie z.B. Kaliumcarbonat, und gegebenenfalls in Gegenwart eines Verdünnungsmittels, wie z.B. Acetonitril, bei Temperaturen zwischen 0 °C und 80 °C umsetzt (vgl. auch die Herstellungsbeispiele).

Die benötigten Ausgangsstoffe der Formeln (IV), (V) und (VI) sind bekannte organische Zwischenprodukte.

Das erfindungsgemäße Verfahren (a) wird unter Einsatz von Alkanolen oder Alkanthiolen oder von deren Alkalimetallsalzen durchgeführt. Vorzugsweise werden Alkanole oder Alkanthiole mit jeweils 1 bis 4 Kohlenstoffatomen, insbesondere mit 1 oder 2 Kohlenstoffatomen, oder deren Natrium- oder Kaliumsalze eingesetzt.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise Aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essig säuremethylester und -ethylester, Nitrile wie z. B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Auch Alkohole, wie z.B. Methanol, Ethanol, Propanol, Isopropanol, Butanol, Isobutanol, sec-Butanol und tert-Butanol können beim erfindungsgemäßen Verfahren (a) als Verdünnungsmittel verwendet werden.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner Aliphatische, aromatische oder heterocyclische Amine, beispielsweise Triethyiamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 20 °C und 120 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erf Erfindungsgemäßen Verfahrens (a) setzt man auf 1 Mol Halogenaryloxynaphthalinderivat der Formel (II) im allgemeinen zwischen 1 und 1000 Mol eines Alkanols oder Alkanthiols und/oder eines Alkalimetallsalzes hiervon ein.

Vorzugsweise werden zwischen 1,5 und 5 Mol eines Alkalimetallsalzes eines Alkanols oder Alkanthiols und zusätzlich zwischen 50 und 500 Mol eines Alkohols je Mol Ausgangsverbindung der Formel (II) eingesetzt.

Die Reaktionskomponenten werden im allgemeinen bei Raumtemperaturen vermischt und das Reaktionsgemisch wird bei erhöhter Temperatur bis zum Ende der Umsetzung gerührt. Die Aufarbeitung kann nach üblichen Methoden erfolgen.

Die beim erfindungsgemäßen Verfahren (b) zur Herstellung von Verbindungen der Formel (I) als Ausgangsstoffe zu verwendenden Aryloxynaphthaline sind durch die Formel (III) allgemein definiert.

In Formel (III) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden und $Y^1$ steht für Sauerstoff oder Schwefel.

Die Ausgangsstoffe der Formel (III) sind noch nicht aus der Literatur bekannt. Man erholt die neuen Aryloxynaphthaline der Formel (III), wenn man Halogenaryloxynaphthaline der allgemeinen Formel (IX)

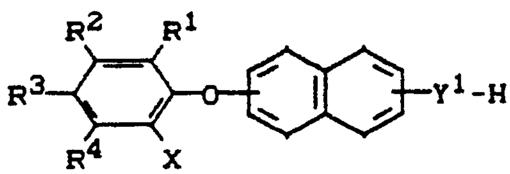

(IX)

in welcher

$R^1$, $R^2$, $R^3$, $R^4$, X und $Y^1$ die oben angegebenen Bedeutungen haben,

mit Alkanolen oder Alkanthiolen, wie z.B. Methanol, Ethanol, Methanthiol oder Ethanthiol und/oder deren Alkalimetallsalzen, vorzugsweise deren Natrium- oder Kaliumsalzen, bei Temperaturen zwischen 20 °C und 120 °C umsetzt (vgl. die Herstellungsbeispiele).

In der Formel (IX) haben $R^1$, $R^2$, $R^3$, $R^4$, X und $Y^1$ vorzugsweise bzw insbesondere diejenigen Bedeutungen, die bereits oben im Zusammenhang mit der Beschreibung der erfindungsgemäßen Verbindungen der Formeln (I), (II) und (III) vorzugsweise bzw. als insbesondere bevorzugt für $R^1$, $R^2$, $R^3$, $R^4$, X und $Y^1$ angegeben wurden.

Die Verbindungen der Formel (IX) sind bekannt und/oder Gegenstand vorgängiger Patentanmeldungen (vgl. EP-A 179 015, EP-A 308 755, EP-A 309 864, EP-A 315 008, DE-P 3 823 318 vom 09.07.1988 oder DE-P 3 821 389 vom 24.06.1988).

Die beim erfindungsgemäßen Verfahren (b) weiter als Ausgangsstoffe zu verwendenden Carbonsäurederivate sind durch die Formel (IV) allgemein definiert. In Formel (IV) haben A und Z vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für A und 2 angegeben wurden und $X^1$ steht vorzugsweise für Chlor, Brom, Jod, für gegebenenfalls durch Fluor oder Chlor substituiertes $C_1$-$C_4$-Alkylsulfonyloxy oder für gegebenenfalls durch Fluor, Chlor, Brom oder Methyl substituiertes Phenylsulfonyloxy, insbesondere für Chlor, Brom, Methylsulfonyloxy, Phenylsulfonyloxy oder 4-Methyl-phenylsulfonyloxy.

Die Ausgangsstoffe der Formel (IV) sind bekannt und/oder können nach an sich bekannten Verfahren hergestellt werden (vgl. DE-OS 2 758 002 und DE-OS 2 854 542).

Das erfindungsgemäße Verfahren (b) wird vorzugsweise in Gegenwart eines Verdünnungsmittels durchgeführt. Es kommen hierbei die gleichen Verdünnungsmittel in Betracht, die oben für das erfindungsgemäße Verfahren (a) angegeben wurden.

Verfahren (b) wird vorzugsweise in Gegenwart eines Säureakzeptors durchgeführt. Es kommen die gleichen Säurebindemittel in Betracht, die oben für das erfindungsgemäße Verfahren (a) angegeben wurden.

Die Reaktionstemperaturen können bei dem erfindungsgemäßen Verfahren (b) in einem größeren Bereich variiert werdend Im allgemeinen arbeitet man bei Temperaturen zwischen 0 °C und 150 °C, vorzugsweise bei Temperaturen zwischen 10 °C und 120 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkrauter der Gattungen: Sinapis, Lepidium, Galium, Stellaria, Matricaria, Anthemis, Galinsoga, Chenopodium, Urtica, Senecio, Amaranthus, Portulaca, Xanthium, eonvoivuius, Spomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitari, Phleum, Poa, Festuca, Eleusine, Brachiaria, Lolium, Bromus, Avena, Cyperus, Sorghum, Agropyron, Cynodon, Monochoria, Fimbristylis, Sagitta ria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Muß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich zur selektiven Unkrautbekämpfung sowohl von monokotylen als auch von dikotylen Unkräutern in monokotylen und dikotylen Kulturen sowohl im Vorauflauf - als auch im Nachauflauf-Verfahren.

Die Wirkstoffe können in die üblichen Formulierungen überführt werden, wie Lösungen, Emulsionen, Spritzpul ver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granuiate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurenäshrstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekampfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]cyclohexancarbonsäure (ALLOXYDIM); 2-Chlor-4-ethylamino-6-isopropylamino-1,3,5-triazin (ATRAZIN); 3-Isopropyl2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); Ethyl-2- {(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYHALID); 2-Chlor-4-ethylamino-6-(3-cyanopropylamino)-1,3,5-triazin (CYANAZIN); 2-[4-(2,4-Dichlorphenoxy)-phenoxyl-propionsäure, deren Methyl- oder deren Ethylester (DICLOFOP); 2-[(2-chlorphenyl)-methyl]-4,4-dimethylisoxazoiidin-3-on (DIMETHAZONE); 4-Amino-6-t-butyl-3-ethylthio-1,2,4-triazin-5(4H)on (ETHIOZIN); 2-[4-C(6-Chlor-2-benzoxazolyl)-oxy]-phenoxy)-propansäure, deren Methyl- oder deren Ethylester (FENOXAPROP); 2-[4-(5-Trifluormethyl-2-pyridyloxy)-phenoxyl-propansäure oder deren Butylester (FLUAZIFOP); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[{3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZSNONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl)-3-chinolincarbonsäure (IMAZAQUIN); 2-[4,5-Dihydro-4-methyl-4-isopropyl-5-oxo-( 1H)-imidazol-2-yl 1-5-ethylpyridin-3-carbonsäure (IMAZEHHAPYR)j 3,5-Diiod-4-hy droxybenzonitril (IOXYNIL); N,N′-Dimethyl-N-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-ehlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); O-(6-Chlor-3-phenyl-pyridazin-4-yl)-S-octyl-thiocarbonat (PYRIDATE); 2-[4-(6-Chlor-chinoxalin-2-yl-oxy)-phenoxy]-propionsaure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäuremethylester (THIAMETURON); N,N-Diisopropyl-S-(2,3,3-trichlorallyl)-thiolcarbamat (THIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmittein ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 5 g und 5000 g Wirkstoff pro Hektar Bodenfläche, vorzugsweise zwischen 10 g und 2000 g pro ha.

Die Herstellung und die Verwendung der erfindungsgemäßen Wirkstoffe geht aus den nachfolgenden Beispielen hervor.

## Herstellungsbeispiele:

### Beispiel 1

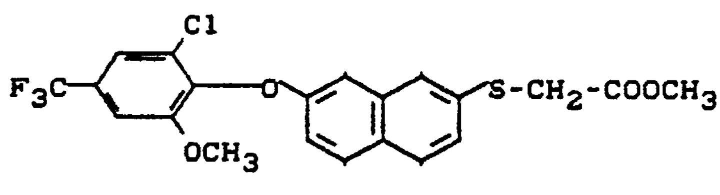

(Verfahren (a))

Eine Mischung aus 8,9 g (0,02 Mol) α-(7-(2-Chlor-6-fluor-4-trifluormethyl-phenoxy)-naphthalin-2-yl-thio)-essigsäuremethylester, 2,7 g (0,05 Mol) Natriummethylat und 200 ml Methanol wird 4 Tage unter Rückfluß erhitzt und dann eingeengt. Der Rückstand wird mit Wasser/Essigsäureethylester ausgeschüttelt, die organische Phase mit Natriumsulfat getrocknet und filtriert. Das Filtrat wird eingeengt und der Rückstand durch Säulenchromatographie (Kieselgel, Toluol) gereinigt.

Man erhält 3,1 g (34% der Theorie) α-(7-(2-chlor-6-methoxy-4-trifluormethyl-phenoxy)-naphthalin-2-yl-thio)-essigsäuremethylester vom Schmelzpunkt 115 °C.

### Beispiel 2

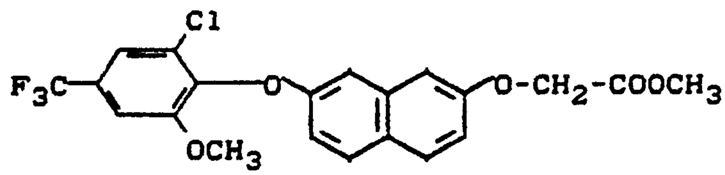

(Verfahren (b))

Eine Mischung aus 1,5 g (4 mMol) 7-(2-Chlor-6-methoxy4-trifluormethyl-phenoxy)-2-naphthol, 0,6 g (4 mMol) Bromessigsäuremethylester, 0,6 g Kaliumcarbonat und 50 ml Acetonitril wird 20 Stunden unter Rückfluß erhitzt und dann eingeengt. Der Rückstand wird mit 2N-Salzsäure/Toluol ausgeschüttelt, die organische Phase mit Natriumsulfat getrocknet und filtriert. Vom Filtrat wird das Lösungsmittel im Wasserstrahlvakuum sorgfältig abdestilliert.

Man erhält 1,8 g (97% der Theorie) α-(7-(2-Chlor-6-methoxy-4-trifluormethyl-phenoxy)-naphthalin-2-yl-oxy)-essigsäuremethylester als amorphen Rückstand.

$^{1}$H-NMR ($CDCl_3$, δ, ppm): 3,80 und 3,81 (OC H 3 und COOC H 3), 4,70 ($CH_2$).

Analog erhalt man:

Beispiel 3

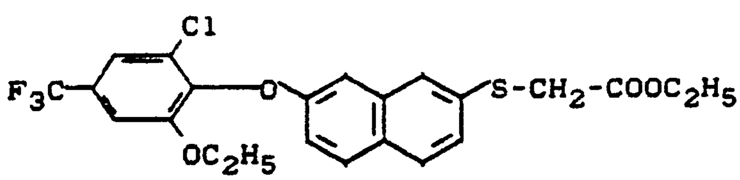

α-(7- (2-Chlor-6-ethoxy-4-trifluormethyl-phenoxy)-naphthalin-2-yl-thio)-essigsäureethylester; Schmelzpunkt: 86 °C.

Beispiel 4

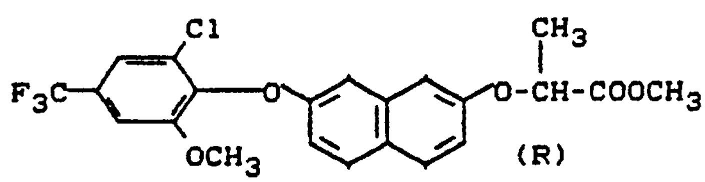

(R)-α-(7-(2-Chlor-6-methoxy-4-trifluormethyl-phenoxy)-naphthalin-2-yl-oxy)-propionsäuremethylester.

$^{1}$H-NMR ($CDCl_3$, δ, ppm): 3,75 und 3,82 (OC H 3 und COOC H 3).

Ausgangsstoffe der Formel (III)

Beispiel (III-1)

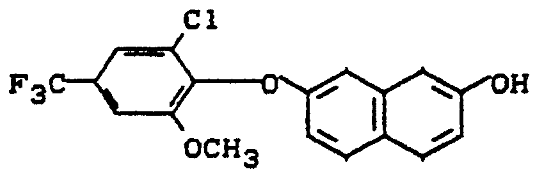

Eine Mischung aus 10,7 g (0,03 Mol) 7-(2-Chlor-6-fluor-4-trifluormethyl-phenoxy)-2-naphthol, 6,4 g (0,12 Mol) Natriummethylat und 150 ml Methanol wird 7 Tage unter Rückfluß erhitzt und dann eingeengt. Der Rückstand wird in Chloroform aufgenommen, mit 1N-Salzsäure und mit Wasser gewaschen, mit Natriumsulfat getrocknet und filtriert. Das Filtrat wird eingeengt, der Rückstand mit Hexan verrieben und das hierbei kristallin angefallene Produkt durch Absaugen isoliert.

Man erhält 5,1 g (46% der Theorie) 7-(2-Chlor-6-methoxy-4-trifluormethyl-phenoxy)-2-naphthol vom Schmelzpunkt 130 °C.

Ausgangsstoffe der Formel (II)

Beispiel (II-1)

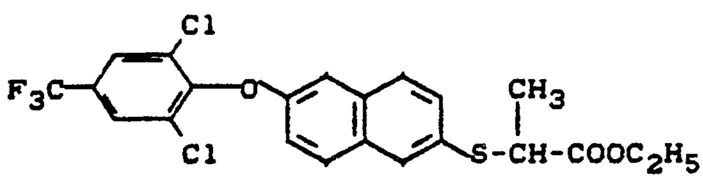

1. Stufe (VII-1):

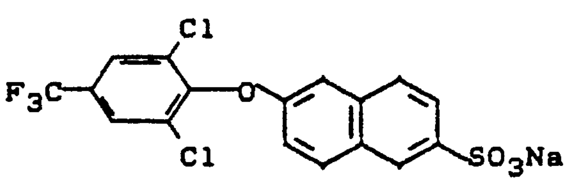

124 g (0,5 Mol) 3,4,5-Trichlorbenzotrifluorid werden unter Rühren zu einer Mischung von 135 g (0,5 Mol) 6-Hydroxy-naphthalin-2-sulfonsäure-Natriumsalz, 30 g (0,54 Mol) Kaliumhydroxid-Pulver und 1000 ml Dimethylsulfoxid gegeben und das Reaktionsgemisch wird zunächst 3 Stunden bei 60 °C und dann 15 Stunden bei 20 °C gerührt. Nach Einengen wird der Rückstand mit Wasser digeriert und das kristallin angefallene Produkt durch Absaugen isoliert. Man erhält 209,5 g (91 % der Theorie) 6-(2,6-Dichlor-4-trifluormethyl-phenoxy)-naphthalin-2-sulfonsäure-Natriumsalz, welches oberhalb 310 °C unter Zersetzung schmilzt.

2. Stufe (VIII-1):

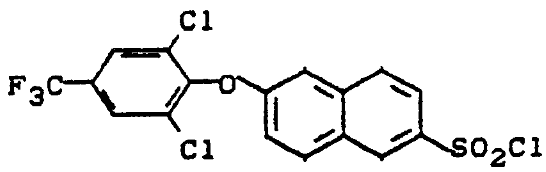

Eine Mischung aus 2,3 g (5,0 mMol) 6-(2,6-Dichlor-4-trifluormethyl-phenoxy)-naphthalin-2-sulfonsäure-Natriumsalz, 1,6 g (7,5 mMol) Phosphor(V)-chlorid und 15 ml Phosphorylchlorid wird zunächst 6 Stunden bei 70° C und dann 15 Stunden bei 20° C gerührt, anschließend in Wasser gegossen und eine weitere Stunde gerührt. Das kristallin angefallene Produkt wird durch Absaugen isoliert.

Man erhält 1,8 g (79 % der Theorie) 6-(2,6-Dichlor-4-trifluormethyl-phenoxy)-naphthalin-2-sulfonsäurechlorid vom Schmelzpunkt 140° C.

3. Stufe (IXa-1)

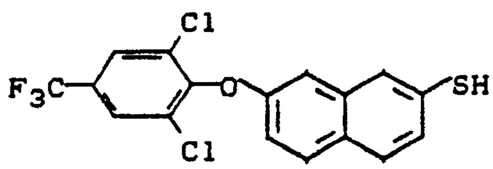

Zu einer Mischung aus 12,5 g (0,19 Mol) Zinkstaub und 100 ml Dioxan werden nacheinander unter Rühren 12,5 g (0,025 Mol) 7-(2,6-Dichlor-4-trifluormethyl-phenoxy)-naphthalin-2-sulfonsaurechlorid und 25 ml konzentrierte Salzsäure gegeben. Das Reaktionsgemisch wird 5 Stunden unter Rückfluß zum Sieden erhitzt und dann weitere 15 Stunden bei 20° C gerührt. Nach Einengen wird der Rückstand in Methylenchlorid/Wasser aufgenommen, filtriert, das Filtrat ausgeschüttelt, die organische Phase abgetrennt, mit Wasser gewaschen, mit Natriumsulfat getrocknet und filtriert.
Das Filtrat wird eingeengt, der Rückstand mit Ethanol digeriert und das hierbei kristallin angefallene Produkt durch Absaugen isoliert.

Man erhält 4,8 g (49 % Theorie) 7-(2,6-Dichlor-4-trifluormethyl-phenoxy)-naphthalin-2-thiol vom Schmelzpunkt 98° C. 4. Stufe (II-1):

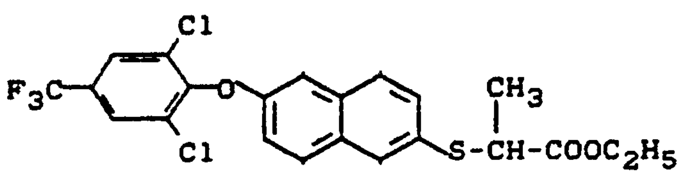

Eine Mischung aus 1,9 g (5 mMol) 6-(2,6-Dichlor-4-trifluormethyl-phenoxy)-naphthalin-2-thiol, 1,0 g (5,5 mMol) α-Brom-propionsäureethylester, 0,7 g (5,5 mMol) Kaliumcarbonat und 50 ml Acetonitril wird 15 Stunden unter Rückfluß zum Sieden erhitzt und anschließend eingeengt. Der Rückstand wird in Methylenchlorid aufgenommen, zweimal mit Wasser gewaschen, mit Natriumsulfat getrocknet und filtriert. Vom Filtrat wird das Lösungsmittel bei 1 Torr (0,13 kPa) sorgfältig abdestilliert.

Man erhält 1,4 g (57 % der Theorie) α-[6-(2,6-Dichlor-4-trifluormethyl-phenoxy)-naphthalin-2-yl-thio]-propionsäureethylester als öligen Rückstand.

$CH_3$

$^1H$-NMR ($CDCl_3$, δ): 3,8 ppm (-C$\underline{H}$-)

Anwendungsbeispiele:

In den nachstehenden Anwendungsbeispielen wird die nachstehend aufgeführte Verbindung (A) als Vergleichssubstanz herangezogen:

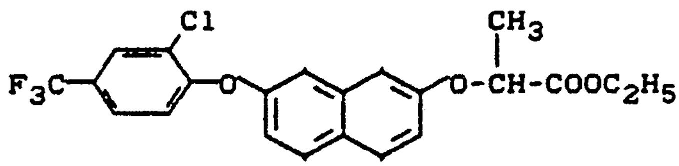

(A)

α-(7-(2-Chlor-4-trifluormethyl-phenoxy)-naphthtalin-2-yl-oxy)-propionsäure-ethylester (bekannt aus EP-A 179015).

Beispiel A

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der herbiziden Wirksamkeit gegenüber der Vergleichssubstanz (A) zeigen in diesem Test z.B. die Verbindungen gemäß den Herstellungsbeispielen 1, 2 und 4.

Beispiel B

Pre-emergence-Test

Lösungsmittel: 5 Gewichsteile Aceton

Emulgator: 1 Gewichtsteil Alkylarylpolyglykolether

Zur Herstellung einsr zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei halt man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffe pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß den Herstellungsbeispielen 1 und 4 starke Wirkung gegen Unkräuter bei guter Verträglichkeit gegenüber Weizen.

## Ansprüche

1. Aryloxynaphthalinderivate dar allgemeinen Formel (I)

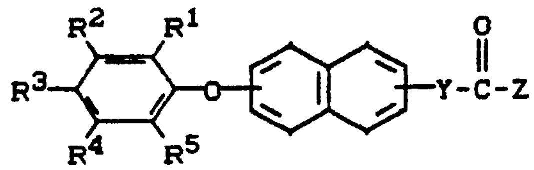

(I)

in welcher

$R^1$ für Wasserstoff, Halogen, Cyano oder Trifluormethyl steht,

$R^2$ für Wasserstoff oder Halogen steht,

$R^3$ für Halogen, Trifluormethyl, Trifluormethoxy, Trifluormethylthio oder Trifluormethylsulfonyl steht,

$R^4$ für Wasserstoff oder Halogen steht,

$R^5$ für Alkoxy oder Alkylthio steht,

Y für eine der Gruppierungen -A-, -O-A- oder -S-A-

steht, wobei

A für gegebenenfalls durch Halogen substituiertes Alkandiyl steht, und

Z für Hydroxy, Alkoxy, Alkenyloxy, Alkinyloxy, Halogenalkoxy, Alkoxyalkoxy, Aralkoxyalkoxy, Alkoxycarbonylalkoxy, Arylalkoxy, Amino, Alkylamino, Alkenylamino, Alkinylamino, Dialkylamino, Dialkenylamino, Dialkinylamino, Alkoxyalkylamino oder Alkoxycarbonylalkylamino steht.

2. Aryloxynaphthalinderivate der Formel (I) gemäß Anspruch 1, in welcher

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Trifluormethyl, Trifluormethoxy, Trifluormethylthio oder Trifluormethylsulfonyl steht,

$R^4$ für Wasserstoff, Fluor oder Chlor steht,

$R^5$ für $C_1$-$C_4$-Alkoxy oder $C_1$-$C_4$-Alkylthio steht,

Y für eine der Gruppierungen -A-, -O-A- oder -S-A-

steht, wobei

A für gegebenenfalls durch Fluor, Chlor und/oder Brom substituiertes $C_1$-$C_4$-Alkandiyl steht, und

Z für Hydroxy, $C_1$-$C_6$-Alkoxy, $C_3$-$C_6$-Alkenyloxy, $C_3$-$C_6$-Alkinyloxy, $C_1$-$C_6$-Halogenalkoxy, $C_1$-$C_4$-Alkoxy-$C_1$-$C_4$-alkoxy, Benzyloxy-$C_2$-$C_3$-alkoxy, $C_1$-$C_4$-Alkoxy-carbonyl-$C_1$-$C_3$-alkoxy, Benzyloxy, Amino, $C_1$-$C_6$-Alkylamino, $C_3$-$C_6$-Alkenylamino, $C_3$-$C_6$-Alkinylamino, Di-($C_1$-$C_4$-alkyl)-amino, Di-($C_3$-$C_4$-alkenyl)-amino, Di-($C_3$-$C_4$-alkinyl)amino, $C_1$-$C_4$-Alkoxy-$C_1$-$C_4$-alkylamino oder $C_1$-$C_4$-Alkoxy-carbonyl-$C_1$-$C_3$-alkylamino steht.

3. Aryloxynaphthalinderivate der Formel (I) gemäß Anspruch 1, in welcher

$R^1$ für Chlor steht,

$R^2$ für Wasserstoff steht,

$R^3$ für Trifluormethyl steht,

$R^4$ für Wasserstoff steht,

$R^5$ für Methoxy, Ethoxy, Propoxy, Methylthio oder Ethylthio steht,

Y für eine der Gruppierungen -$A^1$-, -O-$A^2$- oder -S-$A^2$-

steht, wobei

$A^1$ für gegebenenfalls durch Chlor substituiertes Ethan-1,2-diyl steht und

$A^2$ für Methylen oder Ethan-1,1-diyl (Ethyliden) steht, und

Z für Hydroxy, Methoxy, Ethoxy, Propoxy, Isopropoxy, Butoxy, sec-Butoxy, Isobutoxy, tert-Butoxy, Methoxyethoxy, Ethoxyethoxy, Methoxycarbonylmethoxy, Ethoxycarbonylmethoxy, Methoxycarbonylethoxy, Ethoxycarbonylethoxy, Benzyloxy, Benzyloxyethoxy, Amino, Methylamino, Ethylamino, Propylamino, Isopropylamino, Butylamino, Isobutylamino, sec-Butylamino, tert-Butylamino, Methoxyethylamino, Ethoxyethylamino, Methoxycarbonylmethylamino, Ethoxycarbonylmethylamino, Methoxycarbonylethylamino oder Ethoxycarbonylethylamino steht.

4. Verfahren zur Herstellung von Aryloxynaphthalinderivaten der allgemeinen Formel (I)

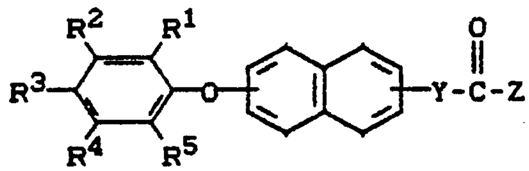

(I)

in welcher

$R^1$ für Wasserstoff, Halogen, Cyano oder Trifluormethyl steht,

$R^2$ für Wasserstoff oder Halogen steht,

$R^3$ für Halogen, Trifluormethyl, Trifluormethoxy, Trifluormethylthio oder Trifluormethylsulfonyl steht,

$R^4$ für Wasserstoff oder Halogen steht,

$R^5$ für Alkoxy oder Alkylthio steht,

Y für eine der Gruppierungen -A-, -O-A- oder -S-A-

steht, wobei

A für gegebenenfalls durch Halogen substituiertes Alkandiyl steht, und

Z für Hydroxy, Alkoxy, Alkenyloxy, Alkinyloxy, Halogenalkoxy, Alkoxyalkoxy, Aralkoxyalkoxy, Alkoxycarbonylalkoxy, Arylalkoxy, Amino, Alkylamino, Alkenylamino, Alkinylamino, Dialkylamino, Dialkenylamino, Dialkinylamino, Alkoxyalkylamino oder Alkoxycarbonylalkylamino steht,

dadurch gekennzeichnet, daß man

(a) Halogenaryloxynaphthalinderivate der allgemeinen Formel (II)

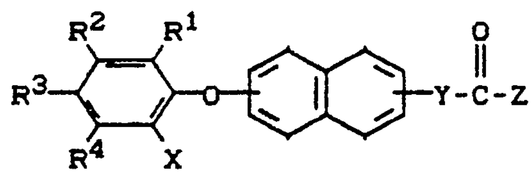

(II)

in welcher

$R^1$, $R^2$, $R^3$, $R^4$, Y und Z die oben angegebenen Bedeutungen haben und

X für Halogen steht,

mit Alkanolen oder Alkanthiolen und/oder mit deren Alkalimetallsalzen gegebenenfalls in Gegenwart eines Säureakzeptors und gegebenenfalls in Gegenwart eines Verdünnungsmittels umsetzt, oder daß man

(b) für den Fall, daß in der Formel (I) Y für eine der Gruppierungen -O-A- oder -S-A- steht und $R^1$, $R^2$, $R^3$, $R^4$, $R^5$ und Z die oben angegebenen Bedeutungen haben,

Aryloxynaphthaline der allgemeinen Formel

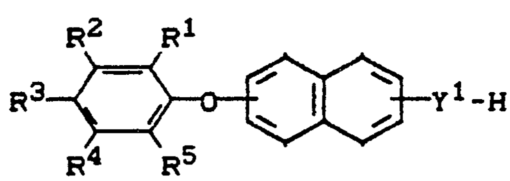

(III)

in welcher

$R^1$, $R^2$, $R^3$, $R^4$ und $R^5$ die oben angegebenen Bedeutungen haben und
$Y^1$ für Sauerstoff oder Schwefel steht,
mit Carbonsäurederivaten der allgemeinen Formel (IV)

$$X^1-A-\overset{\overset{\displaystyle O}{\|}}{C}-Z \qquad (IV)$$

in welcher
A und Z die oben angegebenen Bedeutungen haben und
$X^1$ für eine nucleophile Abgangsgruppe steht,
gegebenenfalls in Gegenwart eines Säureakzeptors und gegebenenfalls in Gegenwart eines Verdünnungsmittels umsetzt.

5. Herbizide Mittel, gekennzeichnet durch einen Gehalt an mindestens einem Aryloxynaphthalinderivat der Formel (I) gemäß Anspruch 1 oder 4.

6. Verfahren zur Bekämpfung von unerwünschten Pflanzen, dadurch gekennzeichnet, daß man Aryloxynaphthalinderivate der Formel (I) gemäß Anspruch 1 oder 4 auf die Pflanzen und/oder ihren Lebensraum einwirken läßt.

7. Verwendung von Aryloxynaphthalinderivaten der Formel (I) gemäß Anspruch 1 oder 4 zur Bekämpfung von unerwünschten Pflanzen.

8. Verfahren zur Herstellung von herbiziden Mitteln, dadurch gekennzeichnet, daß man Aryloxynaphthalinderivate der Formel (I) gemäß Anspruch 1 oder 4 mit Streckmitteln und/oder oberflächenaktiven Substanzen vermischt.

Europäisches Patentamt

# EUROPÄISCHER RECHERCHENBERICHT

Nummer der Anmeldung

EP 90115874.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| D,A | EP - A1 - 0 179 015 (SCHERING) * Ansprüche 1,61-66 * -- | 1,4-8 | C 07 C 69/712<br>C 07 C 323/50<br>A 01 N 39/00 |
| A | EP - A1 - 0 309 864 (BAYER AG) * Ansprüche 1-9 * -- | 1,4-8 | |
| A | EP - A1 - 0 109 311 (STAUFFER CHEMICAL COMPANY) * Ansprüche; Beispiele * -- | 1,4-8 | |
| A | AT - B - 358 557 (CIBA-GEIGY) * Seiten 6-9 * ---- | 1,4-8 | |
| | | | RECHERCHIERTE SACHGEBIETE (Int Cl⁵) |
| | | | C 07 C 59/00<br>C 07 C 69/00<br>C 07 C 235/00<br>C 07 C 323/00 |

Der vorliegende Recherchenbericht wurde für alle Patentansprüche erstellt

| Recherchenort | Abschlußdatum der Recherche | Prüfer |
|---|---|---|
| WIEN | 20-11-1990 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82